# EUROPÄISCHE PATENTSCHRIFT

(11) **EP 2 801 258 B1**
(45) Veröffentlichungstag und Bekanntmachung des Hinweises auf die Patenterteilung: **14.09.2016**
(21) Anmeldenummer: 13167225.5
(22) Anmeldetag: 10.05.2013
(51) Int. Cl.: A22C 11/00, A22C 17/00, A22C 11/02

(54) **Vorrichtung und Verfahren zum Bestimmen von mindestens einem Parameter einer erzeugten Wurst**
Device and method for determining at least one parameter of a manufactured sausage
Dispositif et procédé de détermination d'au moins un paramètre d'une saucisse produite

(43) Veröffentlichungstag der Anmeldung: 12.11.2014
(73) Patentinhaber: Albert Handtmann Maschinenfabrik GmbH & Co. KG, 88400 Biberach (DE)
(72) Erfinder: Fischer, Thomas, 88484 Gutenzell (DE)
(74) Vertreter: Grünecker Patent- und Rechtsanwälte PartG mbB

(56) Entgegenhaltungen:
- EP-A1- 1 570 740
- EP-A2- 1 623 628
- EP-A2- 2 084 969
- DE-A1- 4 307 637

## Beschreibung

Die Erfindung betrifft eine Vorrichtung und ein Verfahren zum Bestimmen von mindestens einem Parameter, der die Wurstform betrifft.

Es sind bereits unterschiedliche Vorrichtungen und Verfahren zum Erfassen der Form von gefüllten Würsten bekannt.

Die EP 1 570 740 A1 beschreibt bereits ein Verfahren und eine Vorrichtung zum Befüllen einer Hülle, wobei Wurstplatzer über eine Abstandsmessung erkannt werden sollen. Um eine Wurstkrümmung bei der Erfassung von Darmplatzern zu kompensieren, sind Abstandssensoren bevorzugt beidseitig einander entgegengesetzt angeordnet. Die Signale der Sensoren werden addiert, so dass sich Zunahme und Abnahme der Abstandssignale ausgleichen.

So ist z.B. allgemein bekannt, Würste die auf einem Transportband befördert werden, optisch mit Kameras im Auflicht- oder Durchlichtverfahren zu erkennen.

Nachteilig bei der Erfassung von Parametern, die die Wurstform betreffen, sind beispielsweise:
- Aufwendiges, empfindliches und teures Equipment,
- hoher Platzbedarf in der Anlage. Die Form von Würsten, die beispielsweise zwischen zwei Transportbändern transportiert werden, kann nicht erfasst werden,
- Störung durch Streulicht,
- Erkennungsrate ist begrenzt,
- Aufwendiges und schwieriges Programmieren und Einteachen der Soll- und Toleranzmaß (Gut-/Schlechtteile).

In der DE 4307637 wird ein Verfahren zum Erkennen der Wurstlänge beschrieben, um die Würste dann voneinander abzutrennen. Mittels eines Lichtstrahls bzw. eines Lichtvorhangs kann dabei der Wurstanfang und das Wurstende und somit die Länge detektiert werden. Der Nachteil bei diesem Verfahren ist, dass keine weiteren Parameter, wie beispielsweise die Krümmung, erfasst werden können, wenn die Würste zwischen zwei Transportbändern gehalten werden.

Hiervon ausgehend liegt der vorliegenden Erfindung die Aufgabe zugrunde, eine verbesserte Vorrichtung und ein verbessertes Verfahren bereitzustellen, die ermöglichen, auf einfache Art und Weise Parameter, die die Form einer erzeugten Wurst betreffen, zu erfassen.

Erfindungsgemäß wird diese Aufgabe durch die Merkmale der Ansprüche 1 und 8 gelöst.

Die erfindungsgemäße Vorrichtung ist geeignet zum Bestimmen von mindestens einem Parameter einer erzeugten Wurst, d.h. einem Parameter, der die Form einer Wurst betrifft, wobei die Vorrichtung als Parameter die Krümmungsrichtung und/oder das Krümmungsmaß bestimmt. Dabei ist eine Transporteinrichtung vorgesehen, insbesondere zwei umlaufende Transportmittel, wie beispielsweise Transportbänder. Die erzeugte Wurst kann dabei in Form von Einzelwürsten oder Wurstketten einer bestimmten Anzahl auf dem Transportmittel befördert werden.

Gemäß der Erfindung weist die Vorrichtung einen Abstandssensor auf, der derart angeordnet ist, dass er einen Abstand zu der Wurst erfassen kann. Ferner ist eine Auswerteeinrichtung vorgesehen, die in Abhängigkeit des Abstands mindestens einen Wurstparameter ermittelt. Mit Hilfe des Abstandssensors kann auf einfache Art und Weise die Oberfläche der sich am Sensor vorbeibewegenden Wurst abgetastet werden, wodurch mehrere Parameter, die die Wurstform betreffen, ermittelt werden können. Wenn der mindestens eine Abstandssensor beispielsweise seitlich von dem Transportmittel angeordnet ist, kann eine Bestimmung der Parameter auch bei Würsten erfolgen, die zwischen umlaufenden Transportmitteln, wie beispielsweise Transportbändern, gefördert werden. Die Erfindung ist also besonders vorteilhaft für das Erfassen der Parameter von Würsten, die zwischen zwei Transportmitteln gehalten werden. Die Vorrichtung eignet sich aber auch für Würste, die auf einem Transportband aufliegen und gefördert werden.

Erfindungsgemäß umfasst die Vorrichtung eine Auswerteeinrichtung, die derart ausgebildet ist, dass in Abhängigkeit des mindestens einen erfassten Parameters ein Signal erzeugt wird, um die Wurst abhängig von diesem Signal entsprechenden Weiterverarbeitungsschritten zuzuführen. Abhängig von bestimmten Parametern können die Würste auch in Wurstgruppen mit entsprechend unterschiedlichen Parametern überführt werden und gegebenenfalls unterschiedlich weiterbehandelt werden.

Vorteilhaft bei der vorliegenden Erfindung ist, dass mittels des mindestens einen Abstandsensors mehrere Parameter erfasst werden können. Dabei wird zusätzlich einer der folgenden Parameter bestimmt:
Portionslänge, Kaliber, Darmplatzer, Vorhandensein einer Trennstelle zwischen aufeinanderfolgenden Wurstportionen.

Beim Ermitteln der Portionslänge kann dann die Auswerteeinheit beispielsweise bestimmen, ob die Länge innerhalb einer bestimmten Vorgabe bzw. Toleranz, insbesondere bei wenig gekrümmten Portionen, liegt. Beim Bestimmen des Kalibers kann dann ermittelt werden, ob der Darm unter- oder überfüllt wird. Beim Ermitteln der Krümmungsrichtung kann bestimmt werden, ob die Würste links oder rechts gekrümmt auf dem Transportmittel liegen. Dieser Parameter kann dann beispielsweise herangezogen werden, um Wurstgruppen mit gleicher Ausrichtung zu bilden. Dies ist für das exakte Ablegen in einer Schale wichtig. Wird das Krümmungsmaß bestimmt, kann beispielsweise daraus die gestreckte Länge errechnet werden. Bei Abweichung kann beispielsweise eine Wurst aus der Produktion ausgeschieden werden. Ferner kann bestimmt werden, ob aufeinanderfolgende Wurstportionen geschnitten oder nicht geschnitten sind. Auch hier kann beispielsweise bei Fehlen einer Trennstelle die entsprechende Wurst bzw. die entsprechenden Würste ausgeschleust werden. Wird als Parameter bestimmt, ob ein Darmplatzer vorliegt, kann ebenfalls eine entsprechende Wurst ausgeschleust werden. Der mindestens eine Abstandssensor ist dabei vorzugsweise ein Reflextaster, insbesondere ein Laserdistanzsensor. Dabei erfolgt die Messung vorzugsweise mit Hilfe von Lasertriangulation oder aber auch anhand einer Laufzeitmessung oder Phasenlagemessung. Aber auch eine Abstandsmessung mit Hilfe von anderen optischen Sensoren, beispielsweise Infrarot-Sensoren ist möglich. Entsprechende Messverfahren sind einfach und kostengünstig und benötigen wenig Platz.

Es ist besonders vorteilhaft, wenn die Messeinrichtung zwei Abstandssensoren umfasst, die auf gegenüberliegenden Seiten der Transporteinrichtung an definierten Stellen angeordnet sind, insbesondere auf gegenüberliegenden Seiten der umlaufenden Transportmittel. Da die Abstandssensoren an definierten Stellen angeordnet sind, und somit deren Abstand bekannt ist, können eine Vielzahl weiterer Parameter exakt bestimmt werden.

Dabei ist vorteilhaft, wenn der mindestens eine Abstandssensor auf einer Höhe angeordnet ist, dass ein Messpunkt P auf einer Ebene liegt, die jeweils den gleichen Abstand zu den beiden umlaufenden Transportmitteln aufweist. Somit ist sichergestellt, dass das exakte Kaliber quer durch eine Wurst bestimmt werden kann. Ferner ist auch sichergestellt, dass der Laserstrahl zur Bestimmung einer Trennstelle auf den sich zwischen zwei Würsten befindenden Wurstzopf gerichtet ist, wenn die Würste nicht voneinander getrennt sind.

Vorteilhafterweise kann der Abstand von beiden umlaufenden Transportmitteln zueinander verstellt werden, so dass auch Würste mit unterschiedlichem Kaliber erzeugt und deren Parameter bestimmt werden können. Es ist jedoch vorteilhaft, dass wenn beispielsweise die Transportmittel aufeinander zu oder voneinander weg bewegt werden, der Mittelpunkt zwischen den Transportmitteln konstant bleibt, derart, dass eine exakte Messung bei unterschiedlichen Parametern möglich ist, ohne dass entsprechende Sensoren umgebaut werden müssen. Dies vereinfacht das Verfahren.

Es ist auch möglich, mehrere Abstandssensoren in einer Richtung übereinander anzuordnen, wobei sich diese Richtung senkrecht zu einer Transportrichtung T der Würste erstreckt. Dies ist insbesondere vorteilhaft, wenn Naturdärme verarbeitet werden, bei denen sich der Wurstzopf zwischen zwei Einzelwürsten nicht immer exakt im Bereich der Mittelachse der Würste befindet. Zusätzlich kann auch zusätzlich zu der Messeinrichtung ein Zeilensensor angeordnet sein, der senkrecht zur Transportrichtung ausgerichtet ist. Über diesen Zeilensensor kann dann eine von der Mittelachse abweichende Abteilstelle aufgefunden werden.

Bei dem Verfahren zum Bestimmen von mindestens einem Parameter, der die Wurstform betrifft, wird die erzeugte Wurst auf einer Transporteinrichtung, insbesondere zwischen zwei umlaufenden Transportmitteln, transportiert und dabei der Abstand von einer definierten Stelle zu der Wurst erfasst und in Abhängigkeit des Abstands mindestens ein Wurstparameter ermittelt.

Es ist vorteilhaft, dass gemäß der Erfindung mit nur einer Vorrichtung mehrere Parameter, die die Form der Wurst betreffen, bestimmt werden können und für weitere Bearbeitungsschritte herangezogen werden können. Abhängig von festgelegten Toleranzen können dann die Einzelwürste oder aber auch eine bestimmte Anzahl zusammenhängender Würste gewünschten Bearbeitungsschritten zugeführt werden.

Es ist ganz besonders vorteilhaft, wenn der Abstand von zwei in Bezug auf das Transportmittel gegenüberliegenden Seiten gemessen wird und der mindestens eine Parameter in Abhängigkeit der beiden Abstände ermittelt bzw. berechnet wird. Somit kann eine noch größere Anzahl von Parametern exakt bestimmt werden, wenn der mindestens eine ermittelte Parameter mit einem Sollparameter oder Sollparameterbereich verglichen wird und in Abhängigkeit des Vergleichs die Wurst einem entsprechenden Weiterverarbeitungsschritt zugeführt wird.

Vorteilhafterweise wird der Abstand in Abhängigkeit der Zeit oder des zurückgelegten Weges der Transporteinrichtung ermittelt. Somit kann beispielsweise mit Hilfe der Messwerte des Abstandssensors bzw. Abstandssensoren und Wegaufnehmern der Transporteinrichtung ein entsprechender Parameter berechnet werden.

Die Wurstlänge als Parameter kann beispielsweise aus der ansteigenden und absteigenden Signalflanke eines Abstandssignals und dem dazwischen zurückgelegten Weg der Transporteinrichtung, insbesondere der Anzahl der Inkremente des Transportmittelantriebs ermittelt werden.

Die Krümmungsrichtung der auf der Transporteinrichtung liegenden Wurst kann auf einfache Art und Weise über die Krümmungsrichtung des Abstandssignals ermittelt werden. Gemäß einer bevorzugten Ausführungsform wird dabei das Abstandssignal in Abhängigkeit der Zeit oder des Weges auf gegenüberliegenden Seiten der Transporteinrichtung zur Wurst ermittelt, wobei bei gekrümmter Wurst der Abstandsignalverlauf auf den gegenüberliegenden Seiten gegenläufig ist, und aus den Abstandssignalen der gegenüberliegenden Seiten die Krümmungsrichtung bestimmt wird. Da bei einer gekrümmten Wurst die Summe der jeweiligen Signale zu einem Zeitpunkt auf den gegenüberliegenden Seiten konstant ist, S₁+S₂=Const, kann die Krümmungsrichtung beispielsweise durch Subtraktion der beiden Messwerte ermittelt werden, indem bestimmt wird, ob das Ergebnis positiv oder negativ ist.

Wenn als Parameter das Wurstkaliber ermittelt wird, wird der Abstand zur Wurst von zwei an definierten Stellen angeordneten in Bezug auf das Transportmittel gegenüberliegenden Abstandssensoren gemessen, wobei der Abstand der Sensoren abzüglich der beiden gemessenen Abstände zur Wurst das ermittelte Kaliber einer nicht gekrümmten Wurst oder das Kaliber einer gekrümmten Wurst in der Wurstmitte ergibt, wobei vorzugsweise das Kaliber an mehreren Stellen der Wurst, vorzugsweise an jeder Stelle der Wurst, bestimmt wird. Somit kann nicht nur das Wurstkaliber selbst bestimmt werden, sondern auch bestimmt werden, ob das Wurstkaliber über die Länge der Wurst konstant ist oder in einem bestimmten Sollbereich liegt.

Bei gekrümmten Würsten wird das ermittelte Kaliber in Abhängigkeit der Krümmung korrigiert.

Als Parameter kann auch das Krümmungsmaß bestimmt werden. Das Krümmungsmaß kann durch Subtraktion des kleinsten gemessenen Abstandssignals vom größten gemessenen Abstandssignal erhalten werden.

Aus dem Parameter Krümmungsmaß und der Länge der gekrümmten Wurst kann die tatsächliche Länge der gestreckten Wurst bestimmt werden.

Gemäß der vorliegenden Erfindung kann ermittelt werden, dass eine Trennstelle zwischen aufeinanderfolgenden Würsten vorliegt, wenn das Abstandssignal auf null absinkt und insbesondere über eine bestimmte Zeit oder Wegstrecke null bleibt oder unterhalb eines vorbestimmten Schwellwertes liegt und insbesondere über eine bestimmte Zeit oder Wegstrecke unterhalb des vorbestimmten Schwellwertes bleibt. Sind zwei aufeinanderfolgende Einzelwürste durchtrennt, gibt es kein Reflexionssignal an dieser Stelle, so dass auf einfache Art und Weise bestimmt werden kann, ob die Würste durchtrennt wurden Es ist aber auch möglich, dass auf Grund eines sehr geringen Wurstabstands, oder auf Grund von zwischen den Würsten liegenden Fleischfasern oder aber Signalrauschen das Abstandsmesssignal nicht ganz auf null absinkt. Dann kann experimentell ein bestimmter Schwellwert festgelegt werden, der als Grundlage zur Bewertung einer Trennstelle dient. Hierbei kann auch der Abstand zwischen zwei aufeinanderfolgenden Würsten ermittelt werden.

Auch ein Darmplatzer kann als Parameter ermittelt werden, beispielsweise wenn es nach einer bestimmten Zeit nach einer aufsteigenden Signalflanke des Abstandssignals keine auf null fallende Signalflanke gibt.

Es ist vorteilhaft, die Krümmungsrichtung der durch die Transporteinrichtung transportierten Würste zu ermitteln und dann Wurstgruppen mit gleicher Krümmungsrichtung zu bilden. In Abhängigkeit der Krümmungsrichtung kann die Wurst beispielsweise auf unterschiedliche weitere Transporteinrichtungen transportiert werden und/oder in verschiedene Aufnahmebehälter eingebracht werden. Das Sortieren der Würste nach ihrer Krümmung ist wesentlich für ein exaktes Ablegen der Würste in Schalen.

Die Erfindung wird im Zusammenhang mit den nachfolgenden Figuren näher erläutert.
- Fig. 1: zeigt grob schematisch eine erste Ausführungsform gemäß der vorliegenden Erfindung.
- Fig. 2a: zeigt grob schematisch eine Seitenansicht einer Füllmaschine mit einer Vorrichtung gemäß der vorliegenden Erfindung.
- Fig. 2b: zeigt eine Aufsicht auf die in Fig. 2a gezeigte Vorrichtung gemäß der vorliegenden Erfindung.
- Fig. 3: zeigt schematisch ein Abstandssignal in Abhängigkeit der Zeit zur Längenerkennung.
- Fig. 4: zeigt schematisch die Abstandssignale in Abhängigkeit der Zeit von zwei Sensoren zur Erkennung der Krümmungsrichtung.
- Fig. 5: zeigt grob schematisch einen Ausschnitt des in Fig. 4 dargestellt Signalverlaufs.
- Fig. 6: zeigt grob schematisch das Krümmungsmaß, das Kaliber und die Wurstlänge.
- Fig. 7: zeigt grob schematisch die Abstandssignalhöhe in Abhängigkeit der Zeit zur Ermittlung, ob eine Trennstelle vorliegt oder nicht.
- Fig. 8: zeigt grob schematisch die Abstandssignalhöhe in Abhängigkeit der Zeit zur Erkennung von Darmplatzern.
- Fig. 9: zeigt grob schematisch einen Querschnitt durch die erfindungsgemäße Vorrichtung sowie unterschiedliche nachfolgende Bearbeitungsschritte.
- Fig. 10: zeigt grob schematisch einen Querschnitt durch die erfindungsgemäße Vorrichtung, wobei eine Wurst ausgeschleust wird.
- Fig. 11: zeigt grob schematisch ein Schaltbild gemäß der vorliegenden Erfindung.
- Fig. 12: zeigt eine weitere mögliche Ausführungsform gemäß der vorliegenden Erfindung mit mehreren übereinander angeordneten Abstandssensoren.

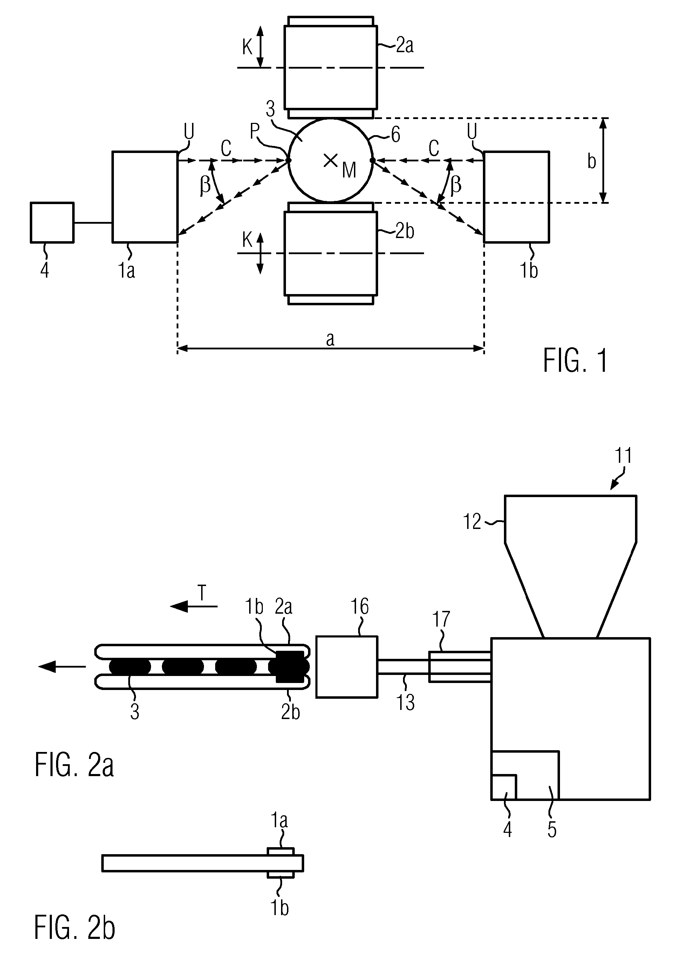

Fig. 1 zeigt eine bevorzugte Ausführungsform gemäß der vorliegenden Erfindung. Die erfindungsgemäße Vorrichtung ist beispielsweise in einer Füllmaschine 11 zum Herstellen von Würsten, wie sie in Fig. 2a gezeigt ist, integriert. Dabei weist eine Füllmaschine 11 einen Trichter 12 zur Aufnahme von pastöser Masse auf, sowie ein integriertes Förderwerk (hier nicht dargestellt), über das die pastöse Masse über ein Füllrohr 13 in eine Wursthülle ausgestoßen wird. Über eine Abdreheinrichtung 17 kann beispielsweise eine Abdrehstelle zum Unterteilen von Würsten hergestellt werden. In Transportrichtung T nach dem Füllrohr kann, wie schematisch durch 16 darstellt ist, beispielsweise eine Abteileinrichtung vorgesehen sein, die Abteilelemente aufweist, die in den befüllten Wurststrang eingreifen und zum Erzeugen einer Abteilstelle die pastöse Masse verdrängen. Zusätzlich kann hier auch noch eine Schneideinrichtung vorgesehen sein, die die abgeteilten Einzelwürste in einzelne Würste oder Wurstgruppen mit mehreren Einzelwürsten abschneidet. Die erzeugten Würste werden über eine Transporteinrichtung 2a,b in Transportrichtung T weitertransportiert. Hier umfasst die Transporteinrichtung zwei umlaufende Transportmittel, beispielsweise zwei umlaufende Transportbänder, zwischen denen die Würste 3 gehalten werden. Die Transportmittel 2a,b können vertikal oder horizontal angeordnet sein. Um Parameter bezüglich der Form der Würste zu erfassen, umfasst die Vorrichtung, wie aus den Fig. 2a und 2b sowie der Fig. 1 hervorgeht, mindestens einen Abstandssensor 1 a,b, in diesem Ausführungsbeispiel gibt es z.B. zwei, gegenüberliegende Abstandssensoren 1 a,b. Wie insbesondere aus Fig. 1 hervorgeht, weisen die beiden Abstandssensoren 1 a, 1 b einen vordefinierten Abstand a auf und sind an vorbestimmten Stellen angeordnet. Die Abstandssensoren 1 a, 1 b sind vorzugsweise derart angeordnet, dass ein Messpunkt P auf einer Ebene liegt, die jeweils den gleichen Abstand zu den beiden Transportmitteln 2a, 2b aufweist, derart, dass beispielsweise der exakte Durchmesser, der durch die Mittelachse M der Wurst 3 hindurchgeht, erfasst werden kann.

Als Abstandssensor wird vorzugsweise ein Reflextaster verwendet. Bei diesem Ausführungsbeispiel wird ein Laserdistanzsensor 1 a, 1 b verwendet, der nach dem Triangulationsprinzip funktioniert. Bei der Lasertriangulation wird ein Laserstrahl, gegebenenfalls auch der Strahl einer Leuchtdiode, auf die sich vorbeibewegende Wurst auf einen Messpunkt P fokussiert und mit einem daneben im Sensor befindlichen Detektor, z.B. einer Kamera, einer ortsauflösenden Fotodiode oder einer CCD-Zeile beobachtet. Ändert sich die Entfernung der Wurst vom Sensor, ändert sich auch der Winkel β unter dem das Licht reflektiert wird und damit auch die Position auf dem Fotoempfänger. Aus der Position des empfangenen reflektierten Strahls wird die Entfernung der Wurstoberfläche vom Sensor berechnet. Somit können die Sensoren 1 a,b die Oberfläche der sich vorbeibewegenden Wurst abtasten. Die Abstandssensoren 1 a,b leiten dann ein dem Abstand c entsprechendes Abstandssignal an eine Auswerteeinrichtung 4. Die Auswerteeinrichtung 4 kann beispielsweise in der Steuerung 5 der Füllmaschine 11 angeordnet sein, kann jedoch auch in einem separaten Steuer- oder Rechnerabschnitt vorgesehen sein. Anstelle des Laserdistanzsensors könnte beispielsweise auch ein anderer optischer Sensor, z. B. ein Infrarot-Sensor zur Abstandsmessung verwendet werden.

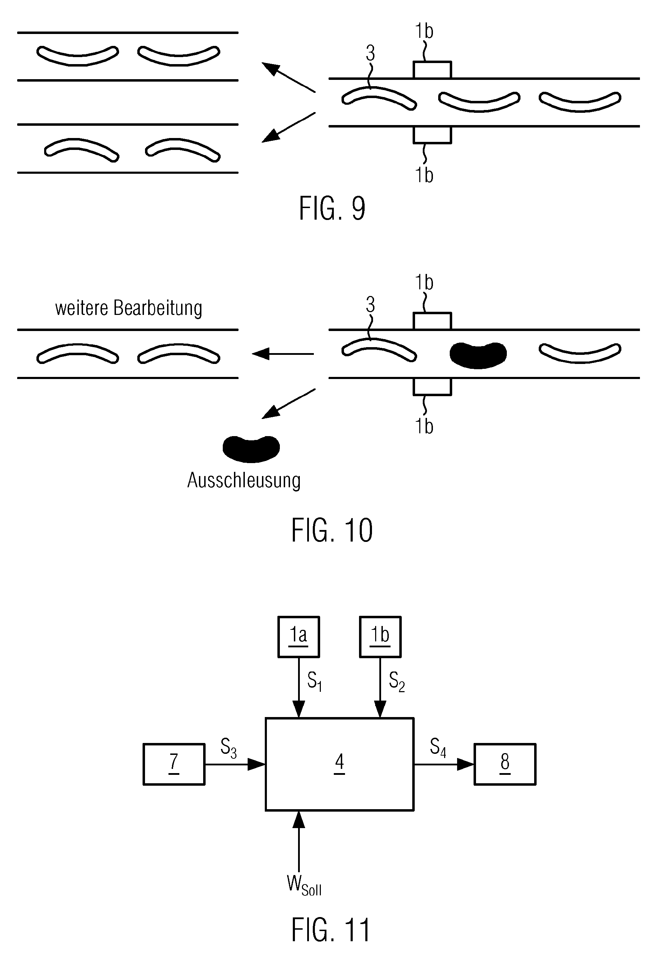

Wie insbesondere aus der Fig. 11 hervorgeht, können die Abstandsmesssignale S1 und S2 der Auswerteeinheit 4 zurückgeführt werden. Ferner kann die Auswerteeinheit 4 beispielsweise auch weitere Signale, beispielsweise von einem Wegaufnehmer 7 der Fördereinrichtung 2a,b empfangen, wobei das Signal S3 dem zurückgelegten Weg der Transporteinrichtung 2a,b pro Zeit entspricht. Das entsprechende Signal S3 kann aber auch von der Steuereinrichtung 5 zugeführt werden. Wie nachfolgend noch näher erläutert wird, kann die Auswerteeinrichtung in Abhängigkeit des Abstands c oder der beiden Abstände c auf gegenüberliegenden Seiten der Transporteinrichtung einen entsprechenden Wurstparameter ermitteln bzw. berechnen, wobei in Abhängigkeit des mindestens einen erfassten Parameters ein Signal S4 erzeugt wird, das an eine Einrichtung 8 weitergeleitet wird, um die Wurst in Abhängigkeit dieses Signals entsprechenden Weiterverarbeitungsschritten zuzuführen. Die Einrichtung 8 kann beispielsweise ein Schieber sein, der die Würste auf verschiedene Transportbänder schiebt oder von der Weiterbearbeitung ausschleust. In die Auswerteeinrichtung 4 können auch entsprechende Sollwerte W_{Soll} oder Sollwertbereiche eingegeben werden, mit denen über eine nicht extra dargestellte Vergleichseinrichtung die ermittelten Parameter verglichen werden können. In Abhängigkeit des Vergleichs wird dann über die Weiterverarbeitung entschieden.

Wie weiter aus der Fig. 1 hervorgeht, weisen die Transportbänder 2a,b einen Abstand b zueinander auf, der im Wesentlichen dem Solldurchmesser bzw. Kaliber der Wurst 3 entspricht. Um auch Würste mit unterschiedlichen Kalibern herzustellen und deren Form erfassen zu können, können die Transportbänder, wie durch den Pfeil K dargestellt ist, aufeinander zu und voneinander weg bewegt werden. Dabei erfolgt die Bewegung der Transportmittel 2a,b derart, dass der Mittelpunkt M zwischen den Transportbändern stets konstant ist, derart, dass eine zwischen den Transportbändern transportierte Wurst stets mit ihrer Mittelachse M im Zentrum zwischen den Transportbändern geführt werden kann. Alternativ kann auch nur ein Transportmittel bewegt werden, wobei dann die Sensoren um die halbe Wegstrecke in gleicher Richtung bewegt werden. Der Messpunkt P liegt dabei auf einer Ebene, die jeweils den gleichen Abstand zu den beiden Transportmitteln aufweist. Mit Hilfe des mindestens einen Abstandsmessers, hier der beiden Abstandsmesser, können vorteilhafterweise mehrere Parameter ermittelt werden. Vorzugsweise wird dabei mindestens einer der folgenden Parameter bestimmt: Portionslänge, Wurstkaliber, Krümmungsrichtung, Krümmungsmaße, Darmplatzer, Vorhandensein einer Trennstelle zwischen aufeinanderfolgenden Würsten.

Nachfolgend wird das erfindungsgemäße Verfahren näher erläutert.

Bei dem erfindungsgemäßen Verfahren wird die erzeugte Wurst auf einer Transporteinrichtung 2a,b, hier zwischen zwei umlaufenden Transportbändern 2a,b, transportiert, wie insbesondere aus den Fig. 2a und 1 hervorgeht. Während die Wurst oder eine Wurstkette mit einer bestimmten Anzahl von Einzelwürsten zwischen den Transportbändern 2a,b in Transportrichtung T gefördert wird, tastet der mindestens eine Abstandssensor 1 a,b, hier die beiden Abstandssensoren, am Messpunkt P die Wurstoberfläche der jeweiligen Wurst ab, während sich die Wurst an den jeweiligen Abstandssensoren 1 a,b vorbeibewegt. Dabei wird der Abstand c zwischen Sensor und Wurst 3 erfasst. In Abhängigkeit dieses gemessenen Abstands bzw. der beiden Abstände kann mindestens ein Parameter ermittelt werden, der die Form der Wurst betrifft. Dabei wird von dem mindestens einen Sensor, hier den beiden Sensoren 1 a,b ein Signal S1, S2 an eine Auswerteinrichtung 4, wie aus Fig. 11 hervorgeht, geleitet. Die Auswerteeinrichtung 4 ermittelt dann einen entsprechenden Parameter. Zur Parameterermittlung kann auch noch von einer Einrichtung 7, beispielsweise einem Wegaufnehmer des Transportmittels, ein weiteres Signal S3 in die Auswerteeinrichtung 4 geleitet werden, wobei das Signals S3 beispielsweise der Geschwindigkeit der Transporteinrichtung entspricht. Der ermittelte Parameter kann dann mit einem Sollwert oder einem Sollwertbereich verglichen werden, wonach ein Signal S4, das von dem Parameter oder dem Vergleich abhängt, ausgegeben wird, das eine Weiterverarbeitungseinrichtung 8 ansteuert, die die entsprechende Wurst einem dem Parameter entsprechenden Weiterverarbeitungsschritt zuführt.

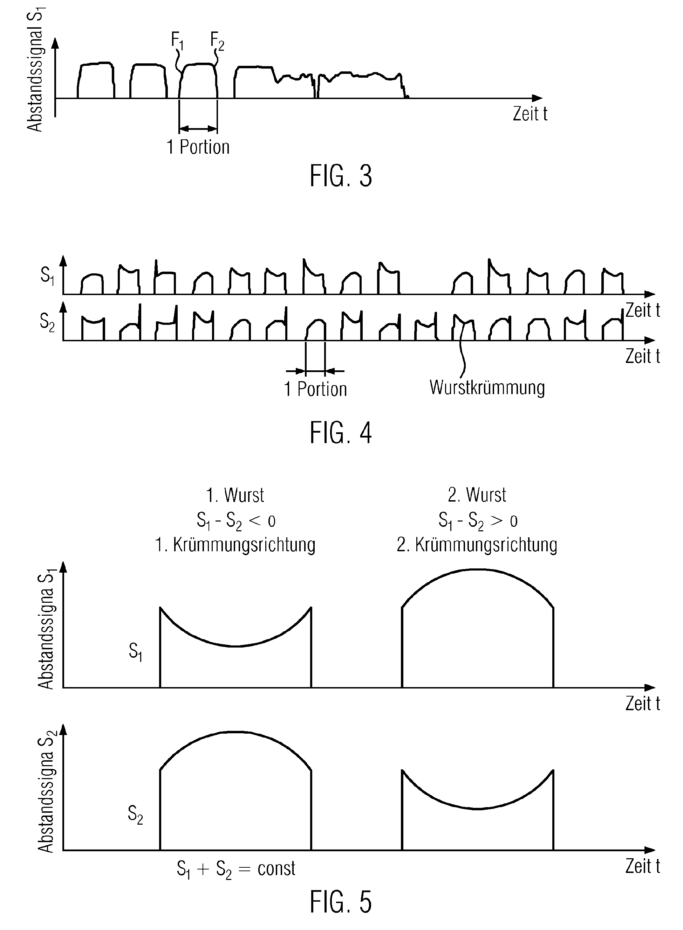

Als Parameter kann beispielsweise die Länge einer Wurst erfasst werden. Dies ist grundsätzlich auch mit nur einem Sensor möglich. Die Fig. 3 zeigt die Abstandssignalhöhe in Abhängigkeit der Zeit oder des Weges. Bewegt sich ein Wurstanfang an dem Abstandssensor 1 a (oder 1 b) vorbei, so wird der Messstrahl reflektiert und das detektierte Abstandssignal S1 steigt an (siehe ansteigende Signalflanke F1). Solange sich die Wurstoberfläche an dem Sensor vorbeibewegt, behält das detektierte Abstandssignal S1 ein hohes Niveau. Am Wurstende wird kein Licht mehr von der Wurstoberfläche reflektiert, so dass das erfasste Abstandssignal S1 auf null absinkt (siehe abfallende Signalflanke F2). Die Länge der Wurst kann aus der ansteigenden und abfallenden Signalflanke F1, F2 und dem zwischen der ansteigenden und der abfallenden Signalflanke zurückgelegten Weg ermittelt werden. Der zurückgelegte Weg kann über die Anzahl der Inkremente des Transportmittelantriebs ermittelt werden.

Ein entsprechendes Signal S3, über das der zurückgelegte Weg ermittelt werden kann, kann beispielsweise von Wegaufnehmern der Transportbänder 7 oder von einer Steuerung 5, beispielsweise in Form der Geschwindigkeit, übermittelt werden.

Ist die Portionslänge bestimmt, wird in der Vergleichseinheit in der Auswerteeinrichtung 4 die Portionslänge mit einem entsprechenden Sollwert oder Sollwertbereich, der vorab eingegeben wurde, verglichen. Liegt die Länge innerhalb der Vorgabe einschließlich Toleranz, kann sie, wie aus Fig. 10 hervorgeht, weiterverarbeitet werden und weiteren Bearbeitungsschritten zugeführt werden. Liegt die Länge nicht in dem entsprechenden Sollwertbereich, so wird die entsprechende Wurst ausgeschleust. Würste unterschiedlicher Länge können unterschiedlich weiterbearbeitet werden.

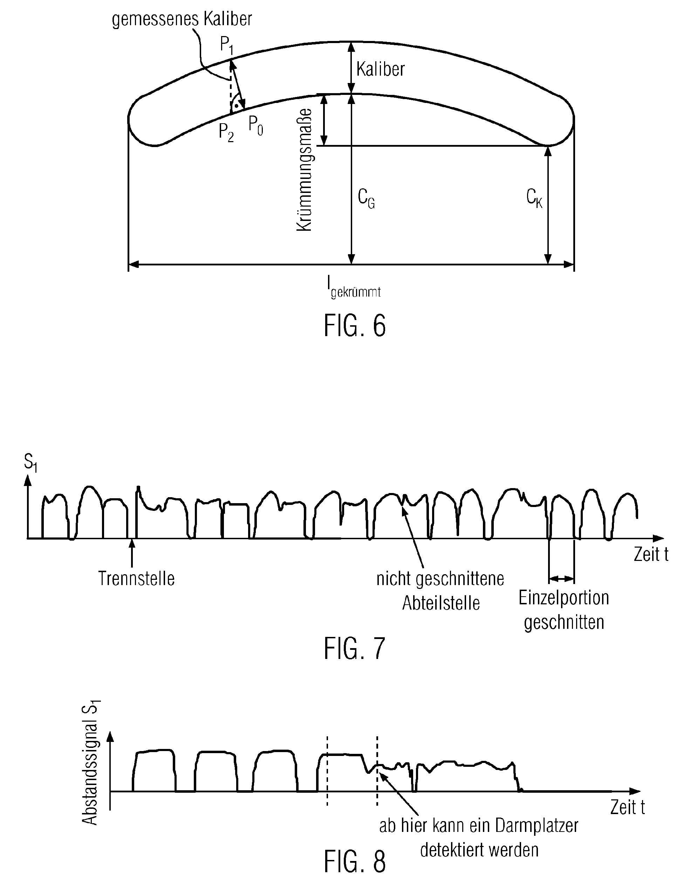

Wenn das Kaliber als Parameter bestimmt werden soll, sind beide Sensoren notwendig. Die beiden Abstandssensoren sind mit definiertem Abstand a zueinander beidseitig der Transportbänder 2a,b montiert. Vom Abstand a der Abstandssensoren 1 a,b werden die beiden gemessenen Abstände c zur Wurst abgezogen, um so das Kaliber (siehe Fig. 6) zu ermitteln.

In der Mitte einer gekrümmten Wurst entspricht das gemessene Kaliber dem tatsächlichen Kaliber der Wurst. Bei gekrümmten Würsten weicht in gekrümmten Bereichen das gemessene Kaliber vom tatsächlichen Kaliber ab, wie aus Fig. 6 hervorgeht. Das tatsächliche Kaliber ist der Durchmesser im wesentlichen senkrecht zur Tangente an einem Punkt auf der gekrümmten Wurstoberfläche. Das gemessene Kaliber kann in Abhängigkeit der ermittelten Krümmung korrigiert werden: Zur Ermittlung des tatsächlichen Kalibers, d.h. bei gekrümmten Würsten der Durchmesser senkrecht zu einer Tangente an einem bestimmten Punkt der Wurst kann beispielsweise trigonometrisch berechnet werden. Zur Ermittlung des tatsächlichen Kalibers kann beispielsweise eine Steigung zwischen zwei Punkten ermittelt werden, beispielsweise zwischen den Punkten P2 und P0 in Fig. 6. Aus den bekannten Strecken P̅1̅,̅ P̅2̅ und P̅2̅,̅ P̅0̅ kann dann das Maß des tatsächlichen Kalibers berechnet werden. Diese Berechnung ist lediglich ein Beispiel für eine mögliche Korrektur.

Durch Bestimmung des Kalibers kann ermittelt werden, ob der Darm unter- oder überfüllt ist. Dabei werden die über die Länge ermittelten Kaliber der Wurst mit entsprechenden Sollwerten bzw. Sollwertbereichen verglichen. Liegt das Kaliber nicht innerhalb der Vorgabe einschließlich Toleranz, so wird die Wurst, wie bereits vorab im Zusammenhang mit Fig. 10 beschrieben ist, ausgeschleust. Die Würste, die innerhalb der Toleranz liegen, können weiter bearbeitet werden. Würste unterschiedlicher Länge können unterschiedlich weiterbearbeitet werden.

Ferner wird als Parameter die Krümmung erfasst.

Wie aus Fig. 4 hervorgeht, weisen gekrümmte Würste ein Abstandssignal auf, das zwischen den aufsteigenden und abfallenden Signalflanken nicht konstant ist sondern konvex oder konkav. Die Krümmungsrichtung konvex oder konkav gibt dann die Krümmungsrichtung der jeweiligen Wurst wieder.

Ganz besonders einfach kann die Krümmungsrichtung mit Hilfe von zwei Sensoren erfasst werden. Bei gekrümmten Würsten ist das Abstandssignal des zweiten Abstandsensors immer gegenläufig zum Abstandssignal des ersten Sensors, sofern die Wurst nicht geplatzt ist. Teilweise auftretende unerwartete Signalspitzen können bei Bedarf ausgefiltert werden. Wie insbesondere aus Fig. 5 hervorgeht, erzeugt beispielsweise ein Abstandssensor 1 a ein Abstandssensorsignal S1 für eine erste Wurst, wobei die Signalhöhe S1 zwischen aufsteigender und abfallender Signalflanke zunächst abnimmt und dann wieder ansteigt. Die Signalhöhe S2 des gegenüberliegenden Sensors verhält sich gegenläufig, derart, dass S₁+S₂=Const. Subtrahiert man nun ein Abstandssignal von einem anderen Abstandssignal, beispielsweise S₁-S₂, ergibt sich hier ein Wert < 0, der eine erste Krümmungsrichtung, hier beispielsweise nach links gekrümmt, anzeigt. Bei einer zweiten Wurst mit einer zweiten Krümmungsrichtung, beispielsweise rechts gekrümmt, ergibt sich S₁-S₂>0.

Ist die Krümmungsrichtung bestimmt, so können dann Wurstgruppen mit gleicher Ausrichtung gebildet werden, wie beispielsweise in Fig. 9 gezeigt ist. Hier werden beispielsweise Würste mit einer ersten Krümmungsrichtung auf eine andere Transporteinrichtung übergeben als Würste mit einer zweiten Krümmung. Würste mit gleicher Krümmungsrichtung können dann exakt in entsprechenden Schalen abgelegt werden.

So kann auf einfache Art und Weise die Krümmungsrichtung bestimmt werden.

Ferner kann als Parameter auch das Krümmungsmaß bestimmt werden. Wie aus Fig. 6 hervorgeht, kann über den Verlauf der Höhe des Abstandssignals während des Durchlaufs einer Wurst durch die Messstelle P das Krümmungsmaß ermittelt werden. In Fig. 6 ergibt sich das Krümmungsmaß durch Subtraktion des kleinsten gemessenen Abstandssignals C_{K} vom größten ermittelten Abstandsmaß C_{G}.

Durch Messen der Abstände an zwei aufeinanderfolgenden Punkte kann jeweils auch eine entsprechende Steigung in einem bestimmten Bereich ermittelt werden.

Aus den beiden Messwerten "gekrümmte Länge" I_{gekrümmt} (Sehnenlänge) und dem Krümmungsmaß lässt sich die tatsächlich gestreckte Länge der Wurst errechnen.

Gemäß der vorliegenden Erfindung kann auch als Parameter bestimmt werden, ob eine Trennstelle vorliegt oder nicht.

Wie aus Fig. 7 hervorgeht, liegt dann eine Trennstelle zwischen zwei aufeinanderfolgenden Würsten 3 vor, wenn das Abstandssignal auf null abfällt, da es zu keiner Reflexion des Messstrahls kommt. Bei dem in Fig. 7 gezeigten Ausführungsbeispiel hängen im vorderen Diagrammabschnitt jeweils zwei Würste 3 aneinander, wobei nach der zweiten Wurst eine Trennstelle erfolgt, und das Signal über einen bestimmten Zeitraum auf null absinkt. Da zwischen den zwei zusammenhängenden Würsten das Signal nicht auf null abfällt, lässt sich daraus schlieβen, dass die Würste zusammenhängen. Am Ende des Diagramms sind geschnittene Einzelportionen gezeigt, bei denen das Abstandssignal nach jeder Wurst wieder auf null abfällt. Somit kann gezielt kontrolliert werden, ob tatsächlich eine Trennstelle vorliegt oder ein Fehler beim Trennen gemacht wurde. Nicht korrekt getrennte Würste oder Wurstketten können dann beispielsweise ausgeschleust werden, wobei die Auswerteeinrichtung als Parameter "nicht ordnungsgemäß getrennt" ermittelt und ein Signal S4 zum Ausschleusen erzeugt.

Als Parameter kann auch bestimmt werden, ob ein Darmplatzer vorliegt.

Wie aus Fig. 8 hervorgeht, erfolgt nach der vierten absteigenden Flanke nach einem vorbestimmten Zeitintervall keine abfallende Signalflanke sondern ein schwankendes Abstandssignal. Ab der gestrichelten Linie kann bestimmt werden, dass ein Darmplatzer vorliegt. Das heißt, wenn nach einer bestimmten Zeit nach einer aufsteigenden Signalflanke des Abstandssignals keine abfallende Signalflanke erfolgt, bestimmt wird, dass ein Darmplatzer vorliegt. Die Auswerteeinrichtung 4 bestimmt dann als Parameter "Darmplatzer" und erzeugt ein Signal S4 zum Ausschleusen der Wurst entsprechend Fig. 10.

Besonders vorteilhaft bei der vorliegenden Erfindung ist, dass mit einer Messanordnung, d.h. mit Hilfe des mindestens einen Abstandsensors, mehrere Parameter, die die Form der Wurst beschreiben, erfasst werden können und abhängig von diesen Parametern entsprechende Weiterverarbeitungsschritte eingeleitet werden können.

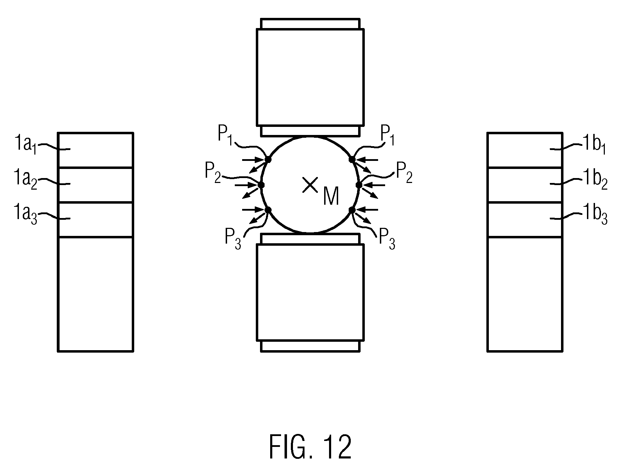

Fig. 12 zeigt eine weitere mögliche Ausführungsform gemäß der vorliegenden Erfindung. Die Ausführungsform entspricht der in Fig. 1 gezeigten Ausführungsform, wobei jedoch hier mehrere Abstandssensoren 1a₁, 1a₂, 1a₃, 1b₁, 1b₂, 1b₃ übereinander seitlich von der Transporteinrichtung angeordnet sind. Somit können mehrere Messpunkte P1, P2, P3 an der Wurstoberfläche erfasst werden. Damit kann beispielsweise sichergestellt werden, dass ein Wurstzopf, der sich z.B. bei Naturdarm, nicht ganz in der Mitte zwischen den Würsten befindet, von der Sensoreinrichtung erfasst werden kann. Alternativ zu dem in Fig. 12 gezeigten Ausführungsbeispiel kann auch zusätzlich zu der in Fig. 1 gezeigten Messeinrichtung senkrecht zur Transportrichtung seitlich neben den Transportbändern ein Zeilensensor angeordnet sein, der das Vorhandensein eines Wurstzopfs erkennt, zum Erkennen einer Trennstelle, die sich gegebenenfalls nicht exakt in der Mitte M befindet.

## Patentansprüche

1. Vorrichtung zum Bestimmen von mindestens einem Parameter, der die Form einer Wurst (3) betrifft, die auf einer Transporteinrichtung (2a,b), insbesondere zwischen zwei umlaufenden Transportmitteln (2a,b), befördert wird, **mit**
einer Messeinrichtung (1a,b), die mindestens einen Abstandssensor (1a,b) umfasst, der derart angeordnet ist, dass er einen Abstand (c) zu einer transportierten Wurst erfassen kann und mit
einer Auswerteeinrichtung (4), die in Abhängigkeit des Abstands (c) mindestens einen Parameter bestimmt, der die Wurstform betrifft, **dadurch gekennzeichnet, dass**
die Auswerteeinrichtung (4) als mindestens einen Parameter die Krümmungsrichtung und/oder das Krümmungsmaß bestimmt und
die Auswerteeinrichtung (4) derart ausgebildet ist, dass sie in Abhängigkeit des mindestens einen erfassten Parameters ein Signal (S4) erzeugt, um die Wurst entsprechenden Weiterverarbeitungsschritten zuzuführen.

2. Vorrichtung nach Anspruch 1 , **dadurch gekennzeichnet, dass** weiter mindestens einer der folgenden Parameter bestimmt wird:
Portionslänge, Kaliber, Darmplatzer, Vorhandensein einer Trennstelle zwischen aufeinanderfolgenden Wurstportionen und insbesondere mehrere Parameter bestimmt werden.

3. Vorrichtung nach mindestens einem der vorherigen Ansprüche, **dadurch gekennzeichnet, dass** der mindestens eine Abstandssensor (1a,b) ein Reflextaster, insbesondere ein Laserdistanzsensor oder Infrarotsensor ist.

4. Vorrichtung nach mindestens einem der vorhergehenden Ansprüche, **dadurch gekennzeichnet, dass** die Messeinrichtung (1a,b) zwei Abstandssensoren (1a,b) umfasst, die auf gegenüberliegenden Seiten der Transporteinrichtung an definierten Stellen angeordnet sind, insbesondere auf gegenüberliegenden Seiten der umlaufenden Transportmittel (2a,b).

5. Vorrichtung nach mindestens einem der Ansprüche 1 bis 4, **dadurch gekennzeichnet, dass** der mindestens eine Abstandssensor (1a,b) auf einer Höhe angeordnet sind, derart, dass ein Messpunkt (P) auf einer Ebene liegt, die jeweils den gleichen Abstand zu den beiden Transportmitteln (2a,b) aufweist.

6. Vorrichtung nach mindestens einem der vorherigen Ansprüche, **dadurch gekennzeichnet, dass** der Abstand (b) beider umlaufender Transportmittel zueinander verstellbar ist, derart, dass der Mittelpunkt (M) zwischen den Transportmitteln konstant bleibt.

7. Vorrichtung nach mindestens einem der vorhergehenden Ansprüche, **dadurch gekennzeichnet, dass** die Messeinrichtung mehrere Abstandssensoren (1a₁, 1b₂, 1a₃, 1b₁, 1b₂, 1b₃) aufweist, die in einer Richtung übereinander, die sich senkrecht zu einer Transportrichtung (T) der Würste erstreckt, angeordnet sind oder wobei zusätzlich ein Zeilensensor vorgesehen ist, der senkrecht zur Transporteinrichtung (T) angeordnet ist.

8. Verfahren zum Bestimmen von mindestens einem Parameter, der die Wurstform betrifft, insbesondere mit einer Vorrichtung nach mindestens einem der Ansprüche 1 bis 7, wobei
die erzeugte Wurst (3) auf einer Transporteinrichtung (2a,b), insbesondere zwischen zwei umlaufenden Transportmitteln (2a,b) transportiert wird,
dabei der Abstand (c) von einer definierten Stelle zu der Wurst erfasst wird und
in Abhängigkeit des Abstands (c) die Krümmungsrichtung und/oder das Krümmungsmaß als Parameter ermittelt wird, wobei
über eine Auswerteeinrichtung (4) in Abhängigkeit des erfassten Parameters ein Signal (S4) erzeugt wird, um die Wurst entsprechenden Weiterverarbeitungsschritten zuzuführen..

9. Verfahren nach Anspruch 8, **dadurch gekennzeichnet, dass** der Abstand (c) von zwei in Bezug auf das Transportmittel (2a,b) gegenüberliegenden Seiten gemessen wird und der mindestens eine Parameter in Abhängigkeit der beiden Abstände ermittelt wird.

10. Verfahren nach mindestens Anspruch 8 oder 9, **dadurch gekennzeichnet, dass** der mindestens eine ermittelte Parameter mit einem Sollparameter oder Sollparameterbereich verglichen wird, und insbesondere in Abhängigkeit des mindestens einen erfassten Parameters ein Signal (S4) erzeugt wird, um die Wurst entsprechenden Weiterverarbeitungsschritten zuzuführen.

11. Verfahren nach mindestens einem der vorhergehenden Ansprüche 8 bis 10, **dadurch gekennzeichnet, dass** der Abstand (c) in Abhängigkeit der Zeit oder des zurückgelegten Wegs der Transporteinrichtung (2a,b) ermittelt wird.

12. Verfahren nach mindestens einem der Ansprüche 8 bis 11, **dadurch gekennzeichnet, dass** als Parameter die Wurstlänge berechnet wird aus einer ansteigenden und einer abfallenden Signalflanke (F1, F2) eines Abstandssignals (S1, S2) und dem dazwischen zurückgelegten Weg des Transportmittels (2a,b), insbesondere der Anzahl der Inkremente des Transportmittelantriebs.

13. Verfahren nach mindestens einem der Ansprüche 8 bis 12, **dadurch gekennzeichnet, dass** der Parameter der Krümmungsrichtung über die Krümmungsrichtung des Abstandsignalverlaufs (S1, S2) ermittelt wird oder wobei der Abstand in Abhängigkeit der Zeit oder des Weges auf gegenüberliegenden Seiten der Transporteinrichtung (2a,b) ermittelt wird, wobei der Abstandssignalverlauf auf den gegenüberliegenden Seiten gegenläufig ist und aus den gegenläufigen Abstandssignalen (S1, S2) die Krümmungsrichtung berechnet wird.

14. Verfahren nach mindestens einem der Ansprüche 8 bis 13, **dadurch gekennzeichnet, dass** als Parameter das Wurstkaliber ermittelt wird, wobei der Abstand (c) zur Wurst von zwei an definierten Stellen gegenüberliegend angeordneten Abstandssensoren gemessen wird, wobei zur Ermittlung des Kalibers vom Abstand (a) der Sensoren die beiden gemessenen Abstände (c) abgezogen werden und insbesondere bei gekrümmten Würsten das ermittelte Kaliber in Abhängigkeit der Krümmung korrigiert wird.

15. Verfahren nach mindestens einem der Ansprüche 8 bis 14, **dadurch gekennzeichnet, dass** als Parameter das Krümmungsmaß bestimmt wird, wobei das Krümmungsmaß durch Subtraktion des kleinsten gemessenen Abstandssignals vom größten gemessenen Abstandssignal erhalten wird.

16. Verfahren nach mindestens einem der Ansprüche 8 bis 15, **dadurch gekennzeichnet, dass** bestimmt wird, dass eine Trennstelle zwischen aufeinanderfolgenden Würsten vorliegt, wenn das Abstandssignal auf null oder unter einen bestimmten Schwellwert absinkt und insbesondere über einen vorbestimmten Zeitraum oder Transportweg der Wurst auf null absinkt oder unterhalb des Schwellwerts bleibt.

17. Verfahren nach mindestens einem der Ansprüche 8 bis 16, **dadurch gekennzeichnet, dass** ein Darmplatzer ermittelt wird, wenn es nach einer bestimmten Zeit nach einer aufsteigenden Signalflanke des Abstandssignals keine abfallende Signalflanke gibt.

18. Verfahren nach mindestens einem der Ansprüche 8 bis 17, **dadurch gekennzeichnet, dass** die Krümmungsrichtung der transportierten Würste ermittelt wird und Wurstgruppen mit gleicher Krümmungsrichtung gebildet werden, wobei die Würste vorzugsweise in Abhängigkeit der Krümmungsrichtung auf verschiedene weitere Transporteinrichtungen transportiert werden und/oder in verschiedene Aufnahmebehälter eingebracht werden.

## Claims

1. An apparatus for determining at least one parameter relating to the shape of a sausage (3), which is transported on a transport device (2a,b), in particular between two circulating means of transport (2a,b) with
a measuring device (1a,b), which comprises at least one distance sensor (1a,b), which is disposed in such a way that said sensor can detect a distance (c) to a sausage that is being transported, and with
an evaluation device (4), which determines at least one parameter relating to the shape of the sausage as a function of the distance (c) **characterized in that,**
the evaluation device (4) determines the direction of curvature and/or the size of curvature as the at least one parameter and
the evaluation device (4) is designed in such a way that a signal (S4) is generated as a function of at least one detected parameter, in order to transport the sausage for corresponding further processing steps.

2. The apparatus according claim 1, **characterized in that** at least one of the following parameters is determined:
Portion length, caliber, split casing, presence of a separation point between successive sausage portions and in particular, a plurality of parameters are determined.

3. The apparatus according to at least one of the preceding claims, **characterized in that** the at least one distance sensor (1a,b) is a reflex scanner, in particular a laser distance sensor or infrared sensor.

4. The apparatus according to at least one of the preceding claims, **characterized in that** the measuring device (1a,b) comprises two distance sensors (1a,b), which are disposed on opposite sides of the transport device at defined location, in particular on opposite sides of the circulating means of transport (2a,b).

5. The apparatus according to at least one of the claims 1 to 4, **characterized in that** the at least one distance sensor (1a,b) is disposed at a height, in such a way that a measuring point (P) lies on a plane, which is at the same distance from each of the two means of transport (2a,b).

6. The apparatus according to at least one of the preceding claims, **characterized in that** the distance (b) of both circulating means of transport relative to one another is adjustable, in such a way that the midpoint (M) between the means of transport remains constant.

7. The apparatus according to at least one of the preceding claims, **characterized in that** the measuring device has a plurality of distance sensors (1a₁, 1b₂, 1a₃, 1b₁, 1b₂, 1b₃), which are disposed one above the other in a direction that extends perpendicular to a transport direction (T) of the sausages, or wherein a line sensor is provided, which is disposed perpendicular to the transport device (T).

8. The method for determining at least one parameter relating to the shape of the sausage, in particular having an apparatus according to at least one of the claims 1 to 7, wherein
the produced sausage (3) is transported on a transport device (2a,b), in particular between two circulating means of transport (2a,b),
in so doing, the distance (c) from a defined location to the sausage is detected and
the direction of curvature and/or size of curvature is determined as parameter as a function of the distance (c),wherein
a signal (S4) is generated as a function of the detected parameter by an evaluation device (4), in order to transport the sausage for corresponding further processing steps

9. The method according to claim 8, **characterized in that** the distance (c) from two sides that are opposite one another with respect to the means of transport (2a,b) is measured, and the at least one Parameter is determined as a function of the two distances.

10. The method according to claim 8 or 9, **characterized in that** the at least one determined parameter is compared to a target parameter or range of target parameters and in particular,
a signal (S4) is generated as a function of the at least one detected parameter, in order to transport the sausage for corresponding further processing steps.

11. The method according to at least one of the preceding claims 8 to 10, **characterized in that** the distance (c) is determined as a function of the time or the distance traveled by the transport device (2a,b).

12. The method according to at least one of the claims 8 to 11, **characterized in that** the length of the sausage is calculated as a parameter from a rising and falling signal edge (F1, F2) of a distance signal (S1, S2), and the distance therebetween traveled by the means of transport (2a,b), in particular the number of increments of the drive on a means of transport.

13. The method according to at least one of the claims 8 to 12, **characterized in that** the parameter of the direction of curvature is determined by means of the direction of curvature of the distance signal characteristic (S1, S2), or wherein the distance is determined as a function of the time or the distance on opposite sides of the transport device (2a,b), wherein the distance signal characteristic on the opposite sides are reversed and the direction of curvature is calculated from opposing distance signals (S1, S2).

14. The method according to at least one of the claims 8 to 13, **characterized in that** the sausage caliber is determined as a parameter, wherein the distance (c) to the sausage from two distance sensors disposed opposite one another at defined locations is measured, wherein in order to determine the caliber from the distance (a) of the sensors, the two measured distances (c) are subtracted and, in particular in the case of curved sausages, the determined caliber is corrected as a function of the curvature.

15. The method according to at least one of the claims 8 to 14, **characterized in that** the size of curvature is determined as a parameter, wherein the size of curvature is obtained by subtracting the smallest measured distance signal from the largest measured distance signal.

16. The method according to at least one of the claims 8 to 15, **characterized in that** it is determined whether a separation point between successive sausages exists, when the distance signal drops to zero or falls below a specific threshold value, and in particular, when the distance signal drops to zero or falls below a specific threshold value for a predetermined period of time or transport distance.

17. The method according to at least one of the claims 8 to 16, **characterized in that** the existence of a split casing is determined when no sinking signal edge occurs after a rising signal edge after a specific period of time.

18. The method according to at least one of the claims 8 to 17, **characterized in that** the direction of curvature of the transported sausage is determined and groups of sausages having the same direction of curvature are formed, wherein the sausages are preferably transported on different, further transport devices depending on the direction of curvature and/or in are placed in different receptacles.

## Revendications

1. Installation destinée à déterminer au moins un paramètre, qui concerne la forme d'une saucisse (3) transportée sur un dispositif de transport (2a,b), notamment entre deux moyens de transport (2a,b) en révolution, comprenant
un dispositif de mesure (1a,b), qui comporte au moins un détecteur de distance (1a,b) agencé de manière à pouvoir détecter et relever une distance (c) à une saucisse transportée, et comprenant
un dispositif de traitement de données (4), qui, en fonction de la distance (c), détermine au moins un paramètre, qui concerne la forme de la saucisse,
**caractérisée en ce que**
le dispositif de traitement de données (4) détermine en tant qu'au moins un paramètre, la direction de courbure et/ou la mesure ou grandeur de la courbure, et
le dispositif de traitement de données (4) est configuré de manière à produire en fonction dudit au moins un paramètre relevé, un signal (S4) destiné à être transmis aux étapes correspondantes poursuivant le traitement des saucisses.

2. Installation selon la revendication 1, **caractérisée en ce que** par ailleurs, on détermine au moins l'un des paramètres suivants :
longueur de portion, calibre, éclatements de boyaux, présence d'une zone de sectionnement entre des portions de saucisse successives, et **en ce que** l'on détermine notamment plusieurs paramètres.

3. Installation selon l'une au moins des revendications précédentes, **caractérisée en ce que** ledit au moins un détecteur de distance (1a,b) est un détecteur à réflexion, notamment un détecteur de distance laser ou un détecteur à infrarouge.

4. Installation selon l'une au moins des revendications précédentes, **caractérisée en ce que** le dispositif de mesure (1a,b) comprend deux détecteurs de distance (1a,b), qui sont agencés sur des côtés opposés du dispositif de transport, en des endroits définis, notamment sur des côtés opposés des moyens de transport (2a,b) en révolution.

5. Installation selon l'une au moins des revendications 1 à 4, **caractérisée en ce que** ledit au moins un détecteur de distance (1a,b) est agencé à une hauteur telle, qu'un point de mesure (P) se situe dans un plan qui présente respectivement la même distance au deux moyens de transport (2a,b).

6. Installation selon l'une au moins des revendications précédentes, **caractérisée en ce que** la distance d'espacement (b) entre les deux moyens de transport en révolution, est réglable de manière à ce que le point médian (M) entre les moyens de transport reste constant.

7. Installation selon l'une au moins des revendications précédentes, **caractérisée en ce que** le dispositif de mesure comprend plusieurs détecteurs de distance (1a₁, 1a₂, 1a₃, 1b₁, 1b₂, 1b₃) qui sont agencés de manière superposée dans une direction s'étendant perpendiculairement à une direction de transport (T), ou en ce qu'il est prévu en supplément un capteur ou détecteur à lignes, qui est agencé perpendiculairement à la direction de transport (T).

8. Procédé pour déterminer au moins un paramètre, qui concerne la forme d'une saucisse, avec notamment une installation selon l'une des revendications 1 à 7, procédé d'après lequel
la saucisse (3) produite est transportée sur un dispositif de transport (2a,b), notamment entre deux moyens de transport (2a,b) en révolution,
on relève la distance (c) d'un endroit défini à la saucisse, et
et on détermine, en fonction de la distance (c), la direction de courbure et/ou la mesure ou grandeur de la courbure en tant que paramètre,
un signal (S4) étant produit par un dispositif de traitement de données (4) en fonction du paramètre relevé, pour être transmis aux étapes correspondantes poursuivant le traitement des saucisses.

9. Procédé selon la revendication 8, **caractérisé en ce que** la distance (c) est mesurée à partir de deux côtés opposés par rapport au moyen de transport (2a,b), et ledit au moins un paramètre est déterminé en fonction des deux distances.

10. Procédé selon au moins la revendication 8 ou la revendication 9, **caractérisé en ce que** ledit au moins un paramètre déterminé est comparé à un paramètre de consigne ou à une plage de paramètre de consigne, et **en ce qu'**on produit notamment un signal (S4) en fonction du paramètre relevé, pour être transmis aux étapes correspondantes poursuivant le traitement des saucisses.

11. Procédé selon l'une au moins des revendications précédentes 8 à 10, **caractérisé en ce que** la distance (c) est déterminée en fonction du temps ou du chemin parcouru par le dispositif de transport (2a,b).

12. Procédé selon l'une au moins des revendications 8 à 11, **caractérisé en ce que** concernant le paramètre, on calcule la longueur de saucisse, à partir d'un flanc de signal montant et descendant (F1, F2) d'un signal de distance (S1, S2) et du chemin parcouru par le moyen de transport (2a,b), notamment le nombre d'incréments de l'entraînement du moyen de transport.

13. Procédé selon l'une au moins des revendications 8 à 12, **caractérisé en ce que** le paramètre de la direction de courbure est déterminé par l'intermédiaire de la direction de courbure du tracé du signal de distance (S1, S2), ou **en ce que** la distance est déterminée en fonction du temps ou du chemin parcouru sur des côtés opposés du dispositif de transport (2a,b), le tracé du signal de distance sur les côtés opposés étant de sens opposé et la direction de courbure étant calculée à partir des signaux de distance (S1, S2) de sens opposés.

14. Procédé selon l'une au moins des revendications 8 à 13, **caractérisé en ce que** concernant le paramètre, on détermine le calibre de la saucisse, on mesure la distance (c) à la saucisse par deux détecteurs ou capteurs de distance agencés en regard l'un de l'autre en des endroits définis, et **en ce que** pour déterminer le calibre, on soustrait de la distance d'espacement (a) des détecteurs ou capteurs, les deux distances (C) mesurées, et notamment dans le cas de saucisses courbes, on corrige le calibre déterminé, en fonction de la courbure.

15. Procédé selon l'une au moins des revendications 8 à 14, **caractérisé en ce que** concernant le paramètre, on détermine la valeur de mesure de la courbure, la valeur de mesure de la courbure étant obtenue par soustraction du plus petit signal de distance mesuré du plus grand signal de distance mesuré.

16. Procédé selon l'une au moins des revendications 8 à 15, **caractérisé en ce que** l'on détermine qu'il existe une zone de sectionnement de séparation entre des saucisses successives, lorsque le signal de distance s'abaisse à zéro ou sous une valeur de seuil déterminée, et notamment s'abaisse à zéro ou reste sous la valeur de seuil pendant une durée prédéterminée ou un chemin de transport déterminé de la saucisse.

17. Procédé selon l'une au moins des revendications 8 à 16, **caractérisé en ce que** l'on détermine un éclatement de boyau, lorsqu'après un temps déterminé à la suite d'un flanc de signal montant du signal de distance, n'apparaît pas de flanc de signal descendant.

18. Procédé selon l'une au moins des revendications 8 à 17, **caractérisé en ce que** l'on détermine la direction de courbure des saucisses transportées et l'on constitue des groupes de saucisses de même direction de courbure, les saucisses étant transportées de préférence en fonction de la direction de courbure, sur d'autres dispositifs de transport différents et/ou sont introduites dans différents récipients de collecte.
